(19) **Europäisches Patentamt / European Patent Office / Office européen des brevets**

(11) **EP 4 559 381 A1**

(12) **EUROPEAN PATENT APPLICATION**
published in accordance with Art. 153(4) EPC

(43) Date of publication:
**28.05.2025 Bulletin 2025/22**

(21) Application number: **23843326.2**

(22) Date of filing: **18.07.2023**

(51) International Patent Classification (IPC):
**A61B 5/00** (2006.01)      **A61B 5/372** (2021.01)
**A61B 5/398** (2021.01)     **G16H 50/20** (2018.01)
**G06N 3/08** (2023.01)

(52) Cooperative Patent Classification (CPC):
**A61B 5/00; A61B 5/372; A61B 5/398; G06N 3/08;
G16H 50/20**

(86) International application number:
**PCT/KR2023/010288**

(87) International publication number:
**WO 2024/019480 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.07.2022 KR 20220089734**

(71) Applicant: **IUCF-HYU (Industry-University
Cooperation
Foundation Hanyang University)
Seoul 04763 (KR)**

(72) Inventors:
• **CHAE, Dong-Kyu**
**Seoul 04763 (KR)**
• **LEE, Harim**
**Seoul 04763 (KR)**
• **SEONG, Eunseon**
**Seoul 04763 (KR)**

(74) Representative: **Schwarz & Partner Patentanwälte
GmbH
Patentanwälte
Wipplingerstraße 30
1010 Wien (AT)**

(54) **METHOD AND SYSTEM FOR TRAINING SELF-SUPERVISED LEARNING BASED-SLEEP STAGE CLASSIFICATION MODEL USING SMALL NUMBER OF LABELS**

(57) Disclosed are a method and system for training a self-supervised learning based-sleep stage classification model using a small number of labels. The sleep stage classification method performed by a computer system according to an embodiment may comprising the steps of: receiving polysomnography data to be inputted into a self-supervised learning based-sleep stage classification model; and classifying sleep stages from the polysomnography data by using the self-supervised learning based-sleep stage classification model, wherein the self-supervised learning based-sleep stage classification model is trained on patterns for sleep stage classification from new sleep data through transfer learning by fine-tuning weights on the basis of a representation learning model that is trained on representations from sleep signal data.

FIG. 1

## Description

## Technical Field

The following description relates to a sleep stage classification technique.

## Background Art

Sleep occupies a huge part of a person's life and significantly affects the body. Since sleep disorders, sleep deprivation, etc. are highly likely to cause problems in our everyday life, such as poor concentration, research on sleep is being actively conducted. Sleep stage classification is an essential step in sleep studies, and the use of artificial intelligence has enabled effective and more accurate prediction. For deep learning-based sleep stage classification, increasing attention is now being paid to approaches employing a supervised learning model, and, more recently, models using self-supervised learning.

However, sleep stage classification methods using a supervised learning model adopt rules by using given labels. These sleep stage classification methods using a supervised learning model have several limitations. There is a practical limit to performing training after producing labels for each dataset to improve the performance of the supervised learning model, and another problem is that the quality of rule learning also depends on the quality of labels. In addition, a sleep stage classification method using a supervised learning model may have performance only on datasets on which training is conducted, which makes the method unfit.

## Disclosure of Invention

## Technical Problem

It is possible to provide a method and system for classifying sleep stages with a small number of labels by extracting latent representations from signals to provide better representations of data through self-supervised learning.

## Solution to Problem

A sleep stage classification method performed by a computer system may comprise the steps of: receiving polysomnography data to be inputted into a self-supervised learning based-sleep stage classification model; and classifying sleep stages from the polysomnography data by using the self-supervised learning based-sleep stage classification model, wherein the self-supervised learning based-sleep stage classification model is trained on patterns for sleep stage classification from new sleep data through transfer learning by fine-tuning weights on the basis of a representation learning model that is trained on representations from sleep signal data.

The self-supervised learning-based sleep stage classification model may be constructed of an unsupervised learning-based adversarial generative model in order to minimize impact on imbalanced class distribution which is inherent in the sleep signal data.

The unsupervised learning-based adversarial generative neural network model may be trained in such a way as to maximize mutual information between a signal generated by a generator and a class code by receiving electroencephalography (EEG) data and electrooculography (EOG) data for a preset time frame extracted from the sleep signal data.

The generator may generate per-class data by receiving a code that represents class identity and noise as an input.

The self-supervised learning-based sleep stage classification model may generate a plurality of positive pairs for the sleep signal data by using an attention-based signal augmentation technique, and may perform pairwise representation learning in such a way as to make the plurality of generated positive pairs similar to each other.

The self-supervised learning-based sleep stage classification model may obtain an attention score for the sleep signal data, and may obtain a plurality of positive pairs with different masks applied thereto by applying a random mask to the obtained attention score and randomly masking out results of an attention score matrix.

The pairwise representation learning may be performed in such a manner as to minimize negative cosine similarity between the generated positive pairs.

The pairwise representation learning may be performed in such a manner as to minimize negative cosine similarity by constructing positive pairs for entire sleep signal data, and in such a manner as to minimize average cosine similarity by constructing positive pairs for a preset signal frame of sleep signal data.

A physical state classification method performed by a computer system may comprise the steps of: receiving biological signal data to be inputted into a self-supervised learning based-physical state classification model; and

classifying physical states from the biological signal data by using the self-supervised learning based-physical state classification model, wherein the self-supervised learning based-sleep stage classification model is trained on patterns for physical state classification from new biological data through transfer learning by fine-tuning weights on the basis of a representation learning model that is trained on representations of biological data.

A computer system for sleep stage classification may comprise: a data input unit that receives polysomnography data to be inputted into a self-supervised learning based-sleep stage classification model; and a sleep stage classification unit that classifies sleep stages from the polysomnography data by using the self-supervised learning based-sleep stage classification model, wherein the self-supervised learning based-sleep stage classification model is trained on patterns for sleep stage classification from new sleep data through transfer learning by fine-tuning weights on the basis of a representation learning model that is trained on representations from sleep signal data.

The self-supervised learning-based sleep stage classification model may be constructed of an unsupervised learning-based adversarial generative model in order to minimize impact on imbalanced class distribution which is inherent in the sleep signal data, and the unsupervised learning-based adversarial generative neural network model may be trained in such a manner as to maximize mutual information between a signal generated by a generator and a class code by receiving EEG data and EOG data for a preset time frame extracted from the sleep signal data.

The self-supervised learning-based sleep stage classification model may generate a plurality of positive pairs for the sleep signal data by using an attention-based signal augmentation technique, and may perform pairwise representation learning in such a way as to make the plurality of generated positive pairs similar to each other.

The self-supervised learning-based sleep stage classification model may obtain an attention score for the sleep signal data, and may obtain a plurality of positive pairs with different masks applied thereto by applying a random mask to the obtained attention score and randomly masking out results of an attention score matrix.

The pairwise representation learning may be performed in such a manner as to minimize negative cosine similarity between the generated positive pairs.

The pairwise representation learning may be performed in such a manner as to minimize negative cosine similarity by constructing positive pairs for entire sleep signal data, and in such a manner as to minimize average cosine similarity by constructing positive pairs for a preset signal frame of sleep signal data.

## Advantageous Effects of Invention

It is possible to accurately classify sleep stages even with a small number of labels by using a self-supervised learning-based sleep stage classification model.

## Brief Description of Drawings

FIG. 1 is a view for explaining a representation learning operation according to an embodiment.
FIG. 2 is a view illustrating an example of data utilization in representation learning and transfer learning according to an embodiment.
FIG. 3 is a block diagram for explaining a configuration of a computer system according to an embodiment.
FIG. 4 is a flowchart for explaining a method for classifying sleep stages according to an embodiment.
FIG. 5 is a view schematically illustrating a method for classifying physical states according to various embodiments of the present disclosure.

## Best Mode for Carrying Out the Invention

Hereinafter, an embodiment will be described in detail with reference to the accompanying drawings.

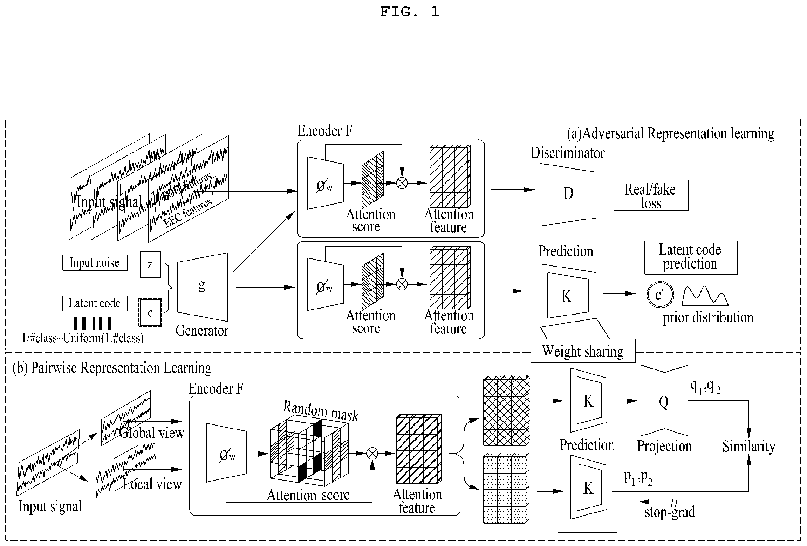

FIG. 1 is a view for explaining a representation learning operation according to an embodiment.

Sleep stage classification refers to classifying a given period of time as a specific stage according to a sleep staging manual on the basis of polysomnographic data. Polysomnography may involve various electrical signal-based polysomnography data recorded during sleep, including various kinds of electroencephalography (EEG) data, electrooculography (EOG) data, electrocardiography (ECG) data, and electromyography (EMG) data. A computer system may automatically classify sleep stages on the basis of polysomnography data. In this case, the computer system may be a sleep stage classification system which classifies sleep stages. The computer system may perform sleep stage prediction with a small number of labels by extracting latent representations from signals to provide better representations of data through a self-supervised learning technique.

The computer system may use attention-based signal augmentation and a self-supervised learning model in order to learn effective representations from EEG data and EOG data which are polysomnography data, and may classify sleep stages with a small number of labels. The computer system may perform a representation learning step in which overall features of polysomnography data are learned using a representation learning model and a transfer learning step in which sleep stage classification is performed with a small number of labels.

The representation learning model may be constructed of a self-supervised learning framework based on a generative model. The representation learning model may extract EEG data and EOG data for a preset time frame (e.g., every 30 seconds (1 epoch)) from polysomnography data for a whole period and use it as input data. In representation learning, an unsupervised learning-based, adversarial generative model is used as a basic framework in order to minimize impact on imbalanced class distribution which is inherent in polysomnography data.

First, the generator may receive a code c that represents class identity and noise z as input data and generate per-class data. In this case, to learn the latent factors for class identification, the adversarial generative model is trained in such a manner as to maximize mutual information between a signal generated by the generator and the class code. In training the adversarial generative model, an objective function for maximizing the mutual information is given by:

Equation 1:

$$\min_{g}\max_{D,F} V_{MI}\ (D,F,G) = V(D,F,G) - \lambda\ I(c;g(c,z))$$

where $V(D,F,g)$ is the loss function of a general adversarial generative model, $D,F,g$ are networks that constitute the adversarial generative model, $D$ is the discriminator, $F$ is the encoder, and $g$ is the generator.

Next, self-supervised learning-based pairwise representation learning may be performed to learn representations of each class. Pairwise representation learning may be performed in such a way as to generate two positive pairs for original signal data (EEG data and EOG data) and make the positive pairs similar to each other. To generate two positive pairs for original sleep signal data, an attention-based signal augmentation technique may be used. First, when encoding an input signal, the attention score may be computed by the following method:

Equation 2:

$$S = \frac{\exp(\text{score}(\emptyset_w(x_k)))}{\sum_{k=1}^{d} \exp(\text{score}(\emptyset_w(x_k)))}$$

where the score denotes inner product computation. After then, a random mask may be applied to the attention score, so that results of an attention score matrix may be randomly masked out in the following way.

Equation 3:

$$z = \emptyset_w(x_k) \cdot (s \odot M)$$

Here, positive pairs $\{Z_1, Z_2\}$ with different masks applied to them may be generated by putting the same sleep signal data (input instance $X_k$) to the encoder $F(\emptyset_w(x_k))$ twice. An overall algorithm for pairwise representation learning is as follows.

Algorithm 1: Positive pairwise representation learning algorithm

| Algorithm 1 SSLAPP's pairwise representation learning. |
| --- |
| Input: EEG and EOG concatenated signals $\{X_1, x_2, ...., x_n\}$ , total number of batch size N, convolution layers $\emptyset_W$, latent predictor K, projector Q, Random mask M. |

(continued)

| Algorithm 1 SSLAPP's pairwise representation learning. |
| --- |

1: for sampled mini batch $\{x_k\}_{k=1}^{N}$ do
2:　　for all $k \in \{1, ..., N\}$ do
3:　　　　#first augmentation
4:　　　　$s_1 = score(\emptyset_w(x_k))$
5:　　　　$z_1 = \emptyset_W(x_K) \cdot (s_1 \odot M)$
6:　　　　#second augmentation
7:　　　　$s_2 = score(\emptyset_w(x_k))$
8:　　　　$z_2 = \emptyset_w(x_K) \cdot (s_2 \odot M)$
9:　　end for
10:　　$p_1 = K(z_1)$ # latent prediction
11:　　$p_2 = K(z_2)$ # latent prediction
12:　　$q_1 = Q(p_1)$ # latent prediction
13:　　$q_2 = Q(p_2)$ # latent prediction
14: end for
15: define the negative similarity loss (5) with $p_1$, $p_2$, $q_1$, $q_2$.

As can be seen from Algorithm 1, learning may be performed in such a manner as to generate two positive pairs by randomly applying a mask to the attention score matrix, and to minimize negative cosine similarity between the positive pairs. The loss function to minimize the negative cosine similarity is given by:

Equation 4:

$$d(p_1, sg(q_2)) = - \frac{<p_1, sg(Q(p_2))>}{\| p_1 \| \| sg(Q(p_2)) \|}$$

After encoding, the two positive pairs are put into a latent prediction model to obtain $p_1$, $p_2$, and the obtained $p_1$, $p_2$ are put into a latent projection model Q to obtain $q_1$, $q_2$. sg denotes the 'stop-gradient' operator that keeps the model's learning parameters from being updated. As a result, when learning latent representations for making two positive pairs similar, the model's learning parameters are updated only for $p_1$, while $q_2$ is used only for obtaining cosine similarity.

In addition, since different sleep stages are shown to have different salient waves within an epoch, learning may be performed by constructing positive pairs for the global loss function and the local loss function, respectively. For the global loss function, learning may be performed in such a way as to minimize negative cosine similarity by constructing positive pairs for an entire signal. The global loss function is given by:

Equation 5:

$$L_{global} = d(p_1^g, sg(q_2^g)) + d(p_2^g, sg(q_1^g))$$

For the local loss function, learning may be performed in such a manner as to minimize average cosine similarity by segmenting a signal into preset time frames (e.g., 5 seconds) and constructing respective positive pairs. The local loss function is given by:

Equation 6:

$$L_{local} = \frac{1}{j} \sum_{i=1}^{j} \{ d(p_1^i, sg(q_2^i)) + d(p_2^i, sg(q_1^i)) \}$$

An objective function for pairwise representation learning including both the local loss function and the global loss function is given by:

$$\text{Equation 7:}$$

$$L_{pos} = \lambda_g \cdot L_{global} + \lambda_l \cdot L_{global}$$

where $\lambda_g$ and $\lambda_l$ are parameters that control the importance of the global loss function and local loss function in the full objective function. Finally, the full objective function including the loss functions of the adversarial generative model and pairwise representation learning is given by:

$$\text{Equation 8:}$$

$$\min_{g,K} \max_{D,F} L_{SSLAPP} = \underbrace{V_{MI}(D,F,g,K)}_{\text{Adversarial loss}} + \lambda_{pos} \underbrace{L_{pos}(K,Q)}_{\text{Pairwise loss}}$$

$\lambda_{oos}$ is a parameter that controls the contribution of pairwise representation learning in the full objective function.

After representation learning, transfer learning may be performed for sleep stage classification. Transfer learning may be conducted by attaching one fully connected layer to the end of a latent projection model Q. Sleep stage classification may be implemented by fine-tuning the weights of a model trained in representation learning and the weights of the one fully connected layer by using a limited number of labels.

Although the embodiment has been described with respect to representation learning and transfer learning operations for sleep stage classification, the present disclosure is not limited by this and the same may apply to physical state classification as well as sleep stage classification.

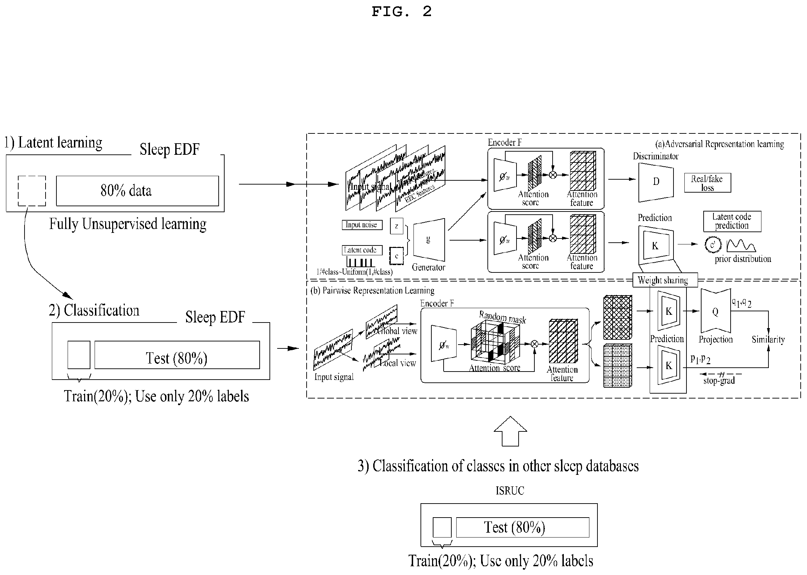

FIG. 2 is a view illustrating an example of data utilization in representation learning and transfer learning according to an embodiment.

A computer system may perform representation learning and transfer learning on the basis of a self-supervised learning model. In the representation learning step, the computer system does not use labels and learns latent representations of data by non-contrastive learning. In the representation learning step, the most frequently used sleep data, Sleep EDF, may be used. In the transfer learning step, the computer system fine-tunes weights on the basis of a representation learning model trained in the representation learning step, and learn sleep stage classification patterns by using a small number of labels. In the transfer learning step, training is done with new data that has not been used in representation learning, whereby the knowledge may be transferred to and used on certain data.

To evaluate the accuracy of sleep stage classification according to the embodiment, Sleep EDFx data and ISRUC data may be used. As for the Sleep EDFx data, 10 % or 20 % of the data that has not been used for sleep data feature learning and its labels may be used to classify sleep stages. As for the ISRUC data, the labels of 10 % or 20 % of ISRUC may be used on a model that is trained on 20 % of the Sleep EDFx data, thereby classifying sleep stages by fine-tuning weights. For performance comparison, average accuracy and F1-macro may be used.

The tables below show the performance of a model for each data type. To compare the performance of sleep stage classification according to the embodiment with conventional methods, a comparison may be made between self-supervised learning-based models using a small number of labels and supervised learning models using all labels.

Table 1: Comparison of performance with self-supervised learning-based model

| Method | Label Ratio | SleepEDF | | ISRUC | |
|---|---|---|---|---|---|
| | | F1-Score | Accuracy | F1-Score | Accuracy |
| RP | 10% | 39.3 ± 2.3 | 62.4 ± 6.0 | 17.1 ± 5.7 | 21.7 ± 7.5 |
| TS | 10% | 38.4 ± 1.9 | 62.5 ± 5.2 | 12.7 ± 2.1 | 21.2 ± 5.4 |
| MoCo | 10% | 24.8 ± 4.1 | 40.9 ± 6.3 | 14.7 ± 3.9 | 27.1 ± 17.2 |
| CPC | 10% | 46.9 ± 6.2 | 65.6 ± 5.4 | 38.6 ± 3.4 | 51.8 ± 4.7 |
| DPC | 10% | 40.1 ± 10.6 | 59.8 ± 11.1 | 32.1 ± 3.2 | 43.9 ± 3.3 |

(continued)

| Method | Label Ratio | SleepEDF | | ISRUC | |
|---|---|---|---|---|---|
| | | F1-Score | Accuracy | F1-Score | Accuracy |
| Triplet Loss | 10% | 37.9 ± 3.4 | 59.4 ± 4.5 | 39.8 ± 3.5 | 49.6 ± 3.1 |
| SleepDPC | 10% | 64.0 ± 1.5 | 70.1 ± 0.8 | 48.9 ± 1.8 | 53.6 ± 1.5 |
| CoSleep | 10% | 55.8 ± 3.0 | 71.6 ± 4.0 | 50.1 ± 5.6 | 57.9 ± 5.1 |
| **SSLAPP** | **10%** | **72.0 ± 0.4** | **77.2 ± 0.3** | **72.5 ± 0.3** | **74.4 ± 0.2** |
| TS-TCC | 20% | 73.5 ± 0.7 | **83.0 ± 0.7** | - | - |
| **SSLAPP** | 20% | **73.9 ± 0.6** | 78.9 ± 0.2 | **74.1 ± 0.3** | **76.1 ± 0.1** |

Table 2: Comparison of performance with supervised learning-based model

| Method | SleepEDF | | ISRUC | |
|---|---|---|---|---|
| | F1-Score | Accuracy | F1-Score | Accuracy |
| SVM | 57.8 | 71.2 | 72.1 | 73.3 |
| RF | 62.4 | 72.7 | 70.8 | 72.9 |
| DeepSleepNet | 75.3 | 78.5 | - | - |
| AttnSleep | 75.1 | 81.3 | - | - |
| **SSLAPP** (**Full label**) | **78.0** | **82.0** | **77.0** | **79.9** |

As can be seen from the experimental results, the method according to the embodiment exhibits outstanding performance with only a small number of labels compared to the conventional methods. Especially, on 10% labels setting, it was observed that the proposed method greatly outperforms other self-supervised learning-based models. It was also observed that the proposed method exhibits outstanding performance on ISRUC data which has not been used in representation learning, compared to the conventional self-supervised and supervised learning-based models. These results show that the proposed model preserves consistency in generalization performance compared to the conventional learning models, and that the proposed model performs relatively accurate sleep stage classification under a limited label setting.

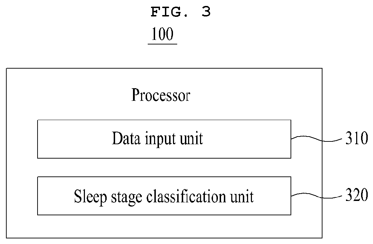

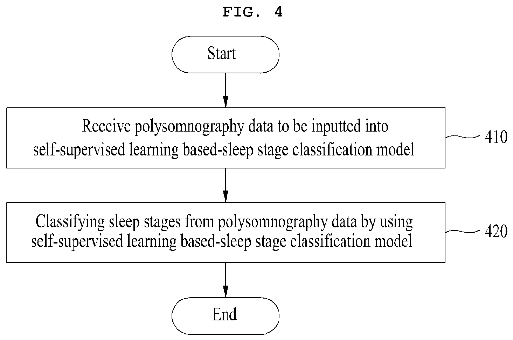

FIG. 3 is a block diagram for explaining a configuration of a computer system according to an embodiment. FIG. 4 is a flowchart for explaining a method for classifying sleep stages according to an embodiment.

A processor of the computer system 100 may include a data input unit 310 and a sleep stage classification unit 320. The components of the processor may be representations of different functions performed by the processor in response to a control instruction provided from a program code stored in the computer system. The processor and the components of the processor may control the computer system to perform the steps 410 and 420 included in the sleep stage classification method of FIG. 4. Here, the processor and the components of the processor may be implemented to execute an instruction in responses to a code of an operating system included in a memory and a code of at least one program.

The processor may load, to the memory, a program code stored in a file of a program for the sleep stage classification method. For example, when the program is executed in the computer system, the processor may control the computer system to load the program code from the file of the program to the memory under control of the operating system. Here, the data input unit 310 and the sleep stage classification unit 320 may be different functional representations of the processor to perform the steps 410 and 420 to be described below by executing an instruction of a part corresponding to the program code loaded to the memory.

In step 410, the data input unit 310 may receive polysomnography data to be inputted into a self-supervised learning-based sleep stage classification model. In this case, polysomnography data of different electrical signals may be inputted into the sleep stage classification model. For example, EEG data and EOG data, which is polysomnography data, may be inputted into the sleep stage classification model.

In step 420, the sleep stage classification unit 320 may classify sleep stages from polysomnography data by using a self-supervised learning-based sleep stage classification model. For example, the sleep stage classification unit 320 may classify sleep stages including a first stage, a second stage, a third stage, and a fifth stage. Alternatively, the sleep stage classification unit 320 may classify sleep stages as a light sleep stage or a deep sleep stage.

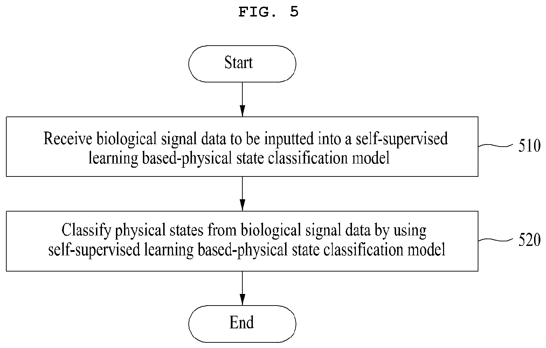

FIG. 5 is a flowchart for explaining a method for classifying physical states.

In step 510, the computer system may receive biological signal data to be inputted into a self-supervised learning-based physical state classification model. In this case, the biological signal data may be result data from a medical checkup or data measured during the user's everyday life.

In step 520, the computer system may classify physical states from biological signal data by using a self-supervised learning-based physical state classification model. For example, a physical state may refer to state information that is distinguished according to the mobility of the user's body. The computer system may classify the user's physical health status based on the user's medical checkup result data of the heart, the brain (stroke, cerebral hemorrhage), the lungs, the kidneys, etc. Alternatively, the computer system may classify the user's physical health status based on (joint) movement data measured during the user's everyday life. In this case, the computer system may classify physical states based on the possible range of motion according to the user's age.

The system described above may be implemented in hardware components, software components, and/or a combination of the hardware components and software components. For example, the system and components described in the embodiments may be implemented using one or more general purpose computers or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions. A processing device may execute an operating system (OS) and one or more software applications running on the operating system. Further, the processing device may access, store, manipulate, process, and generate data in response to execution of the software. For ease of understanding, it has been described that a single processing device is used in some cases, but those skilled in the art will appreciate that the processing device may include a plurality of processing elements and/or a plurality of types of processing elements. For example, the processing device may include a plurality of processors, or a single processor and a single controller. Further, other processing configurations such as a parallel processor are possible.

The software may include a computer program, a code, an instruction, or a combination of one or more thereof, configure the processing device to operate as desired, or independently or collectively command the processing device to operate as desired. The software and/or data may be permanently or temporarily embodied in any type of a machine, a component, a physical device, virtual equipment, a computer storage medium or device, or a signal wave which will be transmitted in order to be interpreted by the processing device or provide a command or data to the processing device. The software may be distributed over computer systems connected through a network and be stored or executed in a distributed manner. The software and data may be stored in one or more computer-readable recording media.

The method according to the embodiment may be implemented in the form of a program instruction which is executable through various computer means and be recorded in a computer-readable medium. The computer-readable medium may include program instructions, data files, data structures, and the like alone or in a combination thereof. The program instructions recorded on the media may be those specially designed and configured for embodiments or may be known and available to those skilled in the art of computer software. Examples of the computer-readable recording medium include magnetic media such as a hard disk, a floppy disk, and a magnetic tape, optical media such as CDROM and DVD, magneto-optical media such as floptical disks, and hardware devices specially configured to store and perform program commands such as read-only memory (ROM), random-access memory (RAM), and flash memory. Examples of the program instructions may include not only machine codes created by a compiler, but also high-level language codes that may be executed by a computer using an interpreter and the like.

Although the embodiments have been described above with reference to the limited embodiments and drawings, those skilled in the art may apply various technical modifications and variations on the basis of the above description. For example, appropriate results may be achieved even when the described technique is performed in a different order from the described method and/or the components of the described system, structure, device, circuit and the like may be combined in a different form or replaced or substituted by other components or equivalents.

Therefore, other implementations, other embodiments, and those equivalents to the claims are within the scope of the claims to be described below.

## Mode for Carrying Out the Invention

The system described above may be implemented in hardware components, software components, and/or a combination of the hardware components and software components. For example, the system and components described in the embodiments may be implemented using one or more general purpose computers or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions. A processing device may execute an operating system (OS) and one or more software applications running on the operating system. Further, the processing device may access, store, manipulate, process, and generate data in response to execution of the software. For ease of understanding, it has been described that a single processing device is used in some cases, but those skilled in the art will appreciate that the processing device may include a plurality of processing elements and/or a plurality of types of processing elements. For example, the processing device may include a plurality of processors, or a single processor and a single controller. Further, other processing configurations such as a parallel processor are possible.

The software may include a computer program, a code, an instruction, or a combination of one or more thereof, configure the processing device to operate as desired, or independently or collectively command the processing device to operate as desired. The software and/or data may be permanently or temporarily embodied in any type of a machine, a component, a physical device, virtual equipment, a computer storage medium or device, or a signal wave which will be transmitted in order to be interpreted by the processing device or provide a command or data to the processing device. The software may be distributed over computer systems connected through a network and be stored or executed in a distributed manner. The software and data may be stored in one or more computer-readable recording media.

The method according to the embodiment may be implemented in the form of a program instruction which is executable through various computer means and be recorded in a computer-readable medium. The computer-readable medium may include program instructions, data files, data structures, and the like alone or in a combination thereof. The program instructions recorded on the media may be those specially designed and configured for embodiments or may be known and available to those skilled in the art of computer software. Examples of the computer-readable recording medium include magnetic media such as a hard disk, a floppy disk, and a magnetic tape, optical media such as CDROM and DVD, magneto-optical media such as floptical disks, and hardware devices specially configured to store and perform program commands such as read-only memory (ROM), random-access memory (RAM), and flash memory. Examples of the program instructions may include not only machine codes created by a compiler, but also high-level language codes that may be executed by a computer using an interpreter and the like.

Although the embodiments have been described above with reference to the limited embodiments and drawings, those skilled in the art may apply various technical modifications and variations on the basis of the above description. For example, appropriate results may be achieved even when the described technique is performed in a different order from the described method and/or the components of the described system, structure, device, circuit and the like may be combined in a different form or replaced or substituted by other components or equivalents.

Therefore, other implementations, other embodiments, and those equivalents to the claims are within the scope of the claims to be described below.

**Claims**

1. A sleep stage classification method performed by a computer system, comprising the steps of:

   receiving polysomnography data to be inputted into a self-supervised learning based-sleep stage classification model; and
   classifying sleep stages from the polysomnography data by using the self-supervised learning based-sleep stage classification model,
   wherein the self-supervised learning based-sleep stage classification model is trained on patterns for sleep stage classification from new sleep data through transfer learning by fine-tuning weights on the basis of a representation learning model that is trained on representations from sleep signal data.

2. The sleep stage classification method of claim 1, wherein the self-supervised learning-based sleep stage classification model is constructed of an unsupervised learning-based adversarial generative model in order to minimize impact on imbalanced class distribution which is inherent in the sleep signal data.

3. The sleep stage classification method of claim 2, wherein the unsupervised learning-based adversarial generative neural network model is trained in such a way as to maximize mutual information between a signal generated by a generator and a class code by receiving electroencephalography (EEG) data and electrooculography (EOG) data for a preset time frame extracted from the sleep signal data.

4. The sleep stage classification method of claim 3, wherein the generator generates per-class data by receiving a code that represents class identity and noise as an input.

5. The sleep stage classification method of claim 1, wherein the self-supervised learning-based sleep stage classification model generates a plurality of positive pairs for the sleep signal data by using an attention-based signal augmentation technique, and performs pairwise representation learning in such a way as to make the plurality of generated positive pairs similar to each other.

6. The sleep stage classification method of claim 5, wherein the self-supervised learning-based sleep stage classification model obtains an attention score for the sleep signal data, and obtains a plurality of positive pairs with different masks applied thereto by applying a random mask to the obtained attention score and randomly masking out results of an attention score matrix.

7. The sleep stage classification method of claim 5, wherein the pairwise representation learning is performed in such a manner as to minimize negative cosine similarity between the generated positive pairs.

8. The sleep stage classification method of claim 5, wherein the pairwise representation learning is performed in such a manner as to minimize negative cosine similarity by constructing positive pairs for entire sleep signal data, and in such a manner as to minimize average cosine similarity by constructing positive pairs for a preset signal frame of sleep signal data.

9. A physical state classification method performed by a computer system, comprising the steps of:

   receiving biological signal data to be inputted into a self-supervised learning based-physical state classification model; and
   classifying physical states from the biological signal data by using the self-supervised learning based-physical state classification model,
   wherein the self-supervised learning based-sleep stage classification model is trained on patterns for physical state classification from new biological data through transfer learning by fine-tuning weights on the basis of a representation learning model that is trained on representations of biological data.

10. A computer system for sleep stage classification, comprising:

    a data input unit that receives polysomnography data to be inputted into a self-supervised learning based-sleep stage classification model; and
    a sleep stage classification unit that classifies sleep stages from the polysomnography data by using the self-supervised learning based-sleep stage classification model,
    wherein the self-supervised learning based-sleep stage classification model is trained on patterns for sleep stage classification from new sleep data through transfer learning by fine-tuning weights on the basis of a representation learning model that is trained on representations from sleep signal data.

11. The computer system of claim 10, wherein the self-supervised learning-based sleep stage classification model is constructed of an unsupervised learning-based adversarial generative model in order to minimize impact on imbalanced class distribution which is inherent in the sleep signal data, and the unsupervised learning-based adversarial generative neural network model is trained in such a way as to maximize mutual information between a signal generated by a generator and a class code by receiving EEG data and EOG data for a preset time frame extracted from the sleep signal data.

12. The computer system of claim 11, wherein the self-supervised learning-based sleep stage classification model generates a plurality of positive pairs for the sleep signal data by using an attention-based signal augmentation technique, and performs pairwise representation learning in such a way as to make the plurality of generated positive pairs similar to each other.

13. The computer system of claim 12, wherein the self-supervised learning-based sleep stage classification model obtains an attention score for the sleep signal data, and obtains a plurality of positive pairs with different masks applied thereto by applying a random mask to the obtained attention score and randomly masking out results of an attention score matrix.

14. The computer system of claim 12, wherein the pairwise representation learning is performed in such a manner as to minimize the negative cosine similarity between the generated positive pairs.

15. The computer system of claim 12, wherein the pairwise representation learning is performed in such a manner as to minimize negative cosine similarity by constructing positive pairs for entire sleep signal data, and in such a manner as to minimize average cosine similarity by constructing positive pairs for a preset signal frame of sleep signal data.

FIG. 1

Encoder F

(a)Adversarial Representation learning

Input signal

local features
EEC features

∅ʹw

Attention score ⊗ Attention feature

Discriminator

D

Real/fake loss

Input noise z

Latent code c

1/#class~Uniform(1,#class)

g

Generator

∅ʹw

Attention score ⊗ Attention feature

Prediction

K

Latent code prediction

c'

prior distribution

Weight sharing

(b) Pairwise Representation Learning

Input signal

Global view

Local view

Encoder F

∅ʹw

Random mask

Attention score ⊗ Attention feature

K

Prediction

Q

Projection

$q_1,q_2$

Similarity

K

$p_1,p_2$

stop-grad

EP 4 559 381 A1

FIG. 2

FIG. 3

100

Processor

| Data input unit | ~ 310 |

| Sleep stage classification unit | ~ 320 |

FIG. 4

```
            ┌─────────────┐
            │    Start    │
            └──────┬──────┘
                   │
                   ▼
┌──────────────────────────────────────────┐
│   Receive polysomnography data to be      │
│   inputted into self-supervised learning  │──── 410
│   based-sleep stage classification model  │
└──────────────────┬───────────────────────┘
                   │
                   ▼
┌──────────────────────────────────────────┐
│  Classifying sleep stages from            │
│  polysomnography data by using            │──── 420
│  self-supervised learning based-sleep     │
│  stage classification model               │
└──────────────────┬───────────────────────┘
                   │
                   ▼
            ┌─────────────┐
            │     End     │
            └─────────────┘
```

FIG. 5

```
                    ┌─────────────┐
                    │    Start    │
                    └─────────────┘
                           │
                           ▼
┌────────────────────────────────────────────────────────┐
│ Receive biological signal data to be inputted into a self-supervised │ ⌐~ 510
│ learning based-physical state classification model      │
└────────────────────────────────────────────────────────┘
                           │
                           ▼
┌────────────────────────────────────────────────────────┐
│ Classify physical states from biological signal data by using │ ⌐~ 520
│ self-supervised learning based-physical state classification model │
└────────────────────────────────────────────────────────┘
                           │
                           ▼
                    ┌─────────────┐
                    │     End     │
                    └─────────────┘
```

## INTERNATIONAL SEARCH REPORT

| International application No. |
| --- |
| **PCT/KR2023/010288** |

| A. | CLASSIFICATION OF SUBJECT MATTER |
| --- | --- |
| | **A61B 5/00**(2006.01)i; **A61B 5/372**(2021.01)i; **A61B 5/398**(2021.01)i; **G16H 50/20**(2018.01)i; **G06N 3/08**(2006.01)i |

According to International Patent Classification (IPC) or to both national classification and IPC

| B. | FIELDS SEARCHED |
| --- | --- |

Minimum documentation searched (classification system followed by classification symbols)

A61B 5/00(2006.01); A61B 5/283(2021.01); A61B 5/369(2021.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수면(sleep), 단계(stage), 분류(classification), 학습(learning), 가중치(weight), 점수 (score)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2022-0082720 A (ASLEEP CO., LTD.) 17 June 2022 (2022-06-17)<br>    See paragraphs [0080]-[0149]. | 1-15 |
| Y | SAEED, Aaqib et al. Federated Self-Supervised Learning of Multisensor Representations for Embedded Intelligence. 15 January 2021. [Retrieved on 16 October 2023]. Retrieved from <IEEE Internet of Things Journal. VOL. 8, NO. 2. URL: https://ieeexplore.ieee.org/abstract/document/9141293>, <DOI: 10.1109/JIOT.2020.3009358>.<br>    See pages 1035-1036. | 1-15 |
| A | WO 2022-020968 A1 (INTERAXON INC.) 03 February 2022.<br>    See claim 1. | 1-15 |
| A | KR 10-2021-0019273 A (HONEYNAPS CO., LTD.) 22 February 2021 (2021-02-22)<br>    See entire document. | 1-15 |
| A | KR 10-2020-0079676 A (HONEYNAPS CO., LTD.) 06 July 2020 (2020-07-06)<br>    See entire document. | 1-15 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **30 October 2023** | **31 October 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/010288**

| Patent document cited in search report | | | Publication date (day/month/year) | Patent family member(s) | | | Publication date (day/month/year) |
|---|---|---|---|---|---|---|---|
| KR | 10-2022-0082720 | A | 17 June 2022 | | None | | |
| WO | 2022-020968 | A1 | 03 February 2022 | CA | 3189784 | A1 | 03 February 2022 |
| | | | | EP | 4189607 | A1 | 07 June 2023 |
| KR | 10-2021-0019273 | A | 22 February 2021 | KR | 10-2258726 | B9 | 15 October 2021 |
| | | | | US | 11464445 | B2 | 11 October 2022 |
| | | | | US | 2021-0045675 | A1 | 18 February 2021 |
| KR | 10-2020-0079676 | A | 06 July 2020 | KR | 10-2267105 | B1 | 22 June 2021 |

Form PCT/ISA/210 (patent family annex) (July 2022)